# EUROPÄISCHE PATENTSCHRIFT

(11) **EP 0 927 759 B1**
(45) Veröffentlichungstag und Bekanntmachung des Hinweises auf die Patenterteilung: **13.10.2004**
(21) Anmeldenummer: 98122350.6
(22) Anmeldetag: 25.11.1998
(51) Int. Cl.: C12N 15/38, C07K 14/04, G01N 33/68, C12Q 1/68, G01N 33/569

(54) **Immunreaktives Protein VP26 des Varicella-Zoster-Virus (VZV) und seine diagnostische Verwendung**
Immunoreactive protein VP26 of Varicella Zoster Virus (VZV) and its diagnostic use
Protéine immunoréactive VP26 du virus Varicella zoster (VZV) et son utilisation diagnostique

(30) Priorität: 23.12.1997 DE 19757765
(43) Veröffentlichungstag der Anmeldung: 07.07.1999
(73) Patentinhaber: Dade Behring Marburg GmbH, 35001 Marburg (DE)
(72) Erfinder: Eickmann, Markus Dr., 35041 Marburg (DE); Gicklhorn, Dorothee, 35075 Gladenbach (DE); Radsak, Klaus Dr. Prof., 35037 Marburg (DE); Hauser, Hans-Peter Dr., 35041 Elnhausen (DE); Giesendorf, Bernard Dr., 35041 Michelbach (DE)

(56) Entgegenhaltungen:
- DAVISON M D ET AL: "Identification of genes encoding two capsid proteins (VP24 and VP26) of herpes simplex virus type 1." JOURNAL OF GENERAL VIROLOGY, Bd. 73, Seiten 2709-13, XP002069558
- DAVISON A J, SCOTT J E: "The complete DNA sequence of varicella-zoster virus." JOURNAL OF GENERAL VIROLOGY, Bd. 67, 1986, Seiten 1759-1816, XP002103724

## Beschreibung

Die vorliegende Erfindung betrifft immunreaktive Peptide, welche dem Bereich der Aminosäurepositionen 12 bis 235 des Proteins VP26 des Varicella-Zoster-Virus homolog sind, dafür kodierende Nukleinsäuren sowie die Verwendung der Peptide oder Nukleinsäuren zur Diagnose einer Varicella-Zoster-Virusinfektion.

Das VZV wird gemäß der Klassifikation des Internationalen Komitees für Virustaxonomie (ICTV) der Familie der Herpesviridae zugeordnet. Die Primärinfektion erfolgt in 75% der Fälle bis zum 15. Lebensjahr und verläuft meist inapparent. Im Gegensatz dazu kann die Infektion von Erwachsenen, die noch keinen Kontakt mit dem Virus hatten, und bei natürlich oder therapeutisch immunsupprimierten Personen mit schwerer Symptomatik verlaufen. Zu ebenfalls schwerer Symptomatik führt die Infektion des Fötus, da das Virus placentagängig ist und zu diesem Zeitpunkt keine Protektion durch maternale Antikörper besteht. Im Anschluß an die Primärinfektion persistiert das Virus lebenslang in sensorischen Ganglien. Nach Reaktivierung breitet sich das VZV über die peripheren Nerven in sensorischen Ganglien aus und führt dann zum Herpes- Zoster.

Aus der vollständig ermittelten Sequenz des VZV-Genoms, mit einer Länge von 124.884 bp, (Stamm Dumas; (A. J. Davison & J. E. Scott. (1986), J. Gen. Virol. 67, 1759-1816)) sind 70 offene Leserahmen (ORF) deduzierbar, darunter auch die offenen Leserahmen der für die derzeit bekannten Glykoproteine gpI (ORF 68), gpII (ORF 31), gpIII (ORF 37), gpIV (ORF 67), gpV(ORF 14) und gpVI (ORF 60). Die aus der Nukleotidsequenz abgeleitete Aminosäuresequenz zeigt jeweils unterschiedlich ausgeprägte Homologien zu den Glykoproteinen gE, gB, gH, gI, gC, und gL des Herpes Simplex Virus (HSV). Es gibt aber keine Anhaltspunkte dafür, daß aus der Sequenzhomologie auch eine Funktionshomologie abgeleitet werden kann. Die offenen Leserahmen der Glykoproteine gpI, gpII, gpIII und gpV konnten molekularbiologisch bestätigt werden.

Die Identifizierung und Charakterisierung von immunogenen Capsidproteinen des VZV beschränkt sich auf wenige Proteine. D. R. Harper und C. Grose ((1989), J. Infect. Dis. 159, 444-451) beschreiben die Immunreaktivität des p32/p36 Komplexes, der dem Nukleocapsid zugeordnet wird. Ferner wird von A. Vafai et al. ((1990), Virus- Res. 15, 163-174) die Kreuzreaktivität humaner VZV-reaktiver Seren beschrieben, die sowohl das Varicella-Zoster-Virus-Nukelocapsidprotein als auch das HSV-Nukleocapsidprotein erkennen. Von keinem der Autoren werden Ansätze vorgeschlagen, die die Verwendung dieser Antigene in diagnostische Testverfahren ermöglichen würden.

Die serologischen Methoden zur Überprüfung der VZV-spezifischen Immunitätslage sind sehr vielfältig und reichen vom Radio- immuno- assay (RIA), Enzyme- linked- immunosorbentassay (ELISA), Fluoreszent- antibody- membran- antigen- assay (FAMA), Immun-Fluoreszenz- Test (IFT) bis zur Komplement- Fixation (CF). Es werden dabei überwiegend VZV spezifische Antikörper nachgewiesen. Als Antigen wird häufig Virusmaterial eingesetzt, welches nach aufwendiger Kultivierung auf humanen Fibroblastenkulturen gewonnen und nach speziellen Methoden für den diagnostischen Einsatz aufbereitet wurde.

Die Gewinnung von VZV-Antigenen aus infizierten Fibroblasten ist für das damit beschäftigte Personal mit einer Infektionsgefahr verbunden. Ferner ist die Herstellung sehr kosten- und zeitaufwendig, unter anderem deswegen, weil das Virus von den infizierten Zellen nicht freigesetzt wird und daher spezielle Reinigungsverfahren erforderlich werden. Für die Verwendung des Antigens ohne vorherige Reinigung für einen immunchemischen Test werden die VZV-infizierten Zellen durch Ultraschallbehandlung aufgeschlossen und das Antigen direkt nach Verdünnung für die Beschichtung von z. B. Mikrotitrationsplatten eingesetzt. Da bei dieser Methode neben virusspezifischen Antigenen überwiegend auch zellspezifische Antigene an die Mikrotitrationsplattenkavitäten gebunden werden, können die zellspezifischen Antigene, besonders beim Vorliegen bestimmter Krankheiten, beispielsweise Autoimmunkrankheiten, zu falsch-positiven Ergebnissen und damit zu Fehldiagnosen führen. Andere Testverfahren, die auf gereinigten viralen Glykoproteinen basieren, wie beispielsweise ein Glykoprotein-ELISA, bedürfen ausgesprochen aufwendiger und verlustreicher Reinigungsverfahren, so daß die Etablierung immunchemischer diagnostischer Tests im größeren Maßstab bisher kaum durchführbar ist. Aufgrund der ausgeprägten Homologie zwischen den Glykoproteinen der α-Herpesviren sind auch für den Glykoprotein-ELISA Kreuzreaktivitäten mit HSV-spezifischen Antikörpern und dadurch bedingt falsch-positive Resultate häufig zu beobachten.

Eine mögliche Lösung der oben aufgeführten Probleme liegt in der Verwendung rekombinanter Proteine, die im heterologen System, z. B. in *Escherichia coli (E. coli),* in großer Menge hergestellt werden können. In diesem System ist eine mögliche VZV-Infektion des Personals ausgeschlossen. Ferner ist die Möglichkeit einer auf spezifische Virusproteine ausgerichteten Differentialdiagnostik gegeben. Insbesondere kann der reaktive Bereich der VZV-Proteine soweit eingegrenzt werden, daß Kreuzreaktivitäten mit für andere Herpesviren spezifischen Antikörpern nahezu oder völlig ausgeschlossen werden können. Die diese Voraussetzungen erfüllenden Proteine können als Hybridproteine exprimiert werden, wobei entweder ein wirtseigenes Protein, beispielsweise das Maltosebindungsprotein (MBP) von *E. coli* oder eine N-terminal gelegene Sequenz aus 6 Histidinresten (His-tag) als Fusionspartner zur Stabilisierung des Expressionsprodukts beitragen. Diese Hybridproteine können dann über Einstufen-Affinitätsreinigungen in annähernd reiner, und damit kontaminationsfreier Form direkt für die Beschichtung von diagnostisch verwendbaren Oberflächen, beispielsweise ELISA-Mikrotitrationsplatten, verwendet werden. Proteine, die die oben genannten Kriterien erfüllen, sind für das VZV-System noch nicht beschrieben worden.

Überraschenderweise konnte ein Teil des ORF23 des VZV als VP26* in E. coli stabil exprimiert werden. Weiterhin wurde überraschenderweise gefunden, daß VP26* immunreaktiv ist und sich in vorteilhafter Weise diagnostisch einsetzen läßt.

Gegenstand vorliegender Erfindung ist somit ein immunreaktives Peptid, welches dem Bereich von AS 12 bis 235 des VP26 des VZV homolog ist oder welches im wesentlichen den Bereich der Aminosäuren 12 bis 235 des VP26 enthält. Ausdrücklich mitumfaßt sollen dabei solche immunreaktive Peptide sein, die aufgrund von VZV-Stammvariationen natürlicherweise variierende Aminosäuresequenzen aufweisen.

Ein weiterer Gegenstand der Erfindung sind Nukleinsäuren, DNS oder RNS, die für die obengenannten erfndungsgemäßen immunreaktiven Peptide kodieren. Insbesondere ist dies die Nukleinsäure mit der Sequenz gemäß Tabelle 1. Darüberhinaus sollen jedoch auch Nukleinsäuren mitumfaßt sein, die mit den vorstehend genannten Nukleinsäuren unter stringenten Bedingungen hybridisieren und für Peptide kodieren, welche von gegen VZV gerichteten Antikörpern, nicht jedoch von gegen andere Herpesviren gerichteten Antikörpern erkannt werden.

Gegenstand der Erfindung sind auch immunreaktive Peptide, die durch Expression der Nukleinsäure mit der Sequenz gemäß Tabelle 1 oder einer der vorstehend genannten, unter stringenten Bedingungen hybridisierenden Nukleinsäuren herstellbar sind, wobei alle Peptide dadurch charakterisiert sind, daß sie von gegen VZV gerichteten Antikörpern, nicht jedoch von gegen andere Herpesviren gerichteten Antikörpern erkannt werden.

Weiterhin ist ein immunchemisches Verfahren Gegenstand der vorliegenden Erfindung, welches den Nachweis von Antikörpern gegen VZV ermöglicht. In diesem Verfahren wird eines oder mehrere der genannten immunreaktiven Peptide mit einer Probe, beispielsweise einer Blut-, Plasma- oder Serumprobe eines Patienten, in Kontakt gebracht, welche auf das Vorhandensein VZV-spezifischer Antikörper untersucht werden soll. Es wird daraufhin mit dem Fachmann geläufigen Methoden festgestellt, ob Antikörper aus der Probe an die eingesetzten immunreaktiven Peptide binden oder mit diesen in eine andere immunologische Wechselbeziehung treten, beispielsweise eine Kompetition.

Die vorliegende Erfindung betrifft außerdem die Verwendung der obengenannten erfindungsgemäßen Nukleinsäure zum Nachweis von VZV mittels Nukleinsäurehybridisierung.

Gegenstand der vorliegenden Erfindung ist darüberhinaus ein Testbesteck zum Nachweis von Antikörpern gegen VZV, enthaltend ein erfindungsgemäßes immunreaktives Peptid sowie ein Testbesteck zum Nachweis von VZV, enthaltend die erfindunggemäße Nukleinsäure.

### Detaillierte Beschreibung der Erfindung:

Zur Lokalisation diagnostisch verwendbarer immunreaktiver Bereiche wurde eine Definition immunreaktiver Proteine des VZV mittels Immunscreening einer genomischen VZV-Bibliothek durchgeführt. Dazu wurde eine genomische VZV-Bibliothek als Phagenbank im Zap-Express-System (Stratagene) angelegt. Mit dieser Bibliothek wurde ein Immunoscreening unter Verwendung eines Pool-Serums aus 25 VZV-reaktiven Humanseren durchgeführt. Die DNS positiv bewerteter Klone wurde in die zirkuläre Form überführt, sequenziert und dem entsprechenden Bereich der VZV-Sequenz zugeordnet. Anhand dieser Methode konnte ein immunreaktiver Klon isoliert werden, der den ORF23 von AS12 - 235 exprimierte. Für diesen Leserahmen wurde im VZV-System noch kein Expressionsprodukt beschrieben. Aus Homologiestudien zwischen VZV und HSV ist jedoch bekannt, daß der ORF23 dem UL36 des HSV1 entspricht (A. J. Davison & J. E. Scott. (1986), J. Gen. Virol. 67, 1759-1816). Das entsprechende Genprodukt des Herpes Simplex Virus ist jedoch mit 116 AS erheblich kürzer und zeigt auch keine ausgesprochene Homologie. Weder für das VZV ORF-23 Genprodukt noch für das HSV UL36-Genprodukt konnte bisher eine Immunreaktivität (von Humanen-Seren) nachgewiesen werden. Aufgrund der oben aufgeführten Ergebnisse stellt das Genprodukt des ORF23 (VP26) einen möglichen Kandidaten für ein diagnostisches Testverfahren dar. Infolgedessen wurde sowohl der gesamte ORF23 als auch der N-terminal verkürzte Bereich in den Vektor pMAL-c2 subkloniert. Daraus resultierten die Konstrukte pMAL-VP26 und pMAL-VP26*. Diese wurden in überlappender, bidirektionaler Weise sequenziert. Die Sequenz weist keine Veränderungen im Vergleich mit der publizierten Sequenz (A. J. Davison & J. E. Scott. (1986), J. Gen. Virol. 67, 1759-1816) auf. Die Expression dieser Konstrukte ergab, daß das Gesamtprotein im Gegensatz zu dem verkürzten Protein nicht stabil exprimiert werden konnte. Die Immunreaktivität des pMAL-VP26*-Produkts wurde mit dem Pool-Serum (s. o.) als positiv bewertet. Auch im Anschluß an die Subklonierung des VZV-spezifischen DNS-Fragments aus dem Vektor pMAL-VP26* in den Vektor pQE30 und Expression im pQE-System konnte die Immunreaktivität bestätigt werden. Somit konnte eine mögliche falsch-positive Einschätzung, bedingt durch den MBP-Fusionsanteil, ausgeschlossen werden.

Bei einer Bewertung im ELISA konnte bei pMAL-VP26* mit einem errechneten cut off = 187 (Mittelwert der Negativseren + 3mal die Standardabweichung) eine Sensitivität von 69% (n=13) und eine Spezifität von 98% (n=41) bzw. mit einem frei gewählten cut off von 120 eine Sensitivität von 85% und eine Spezifität von 95% für die IgM-Diagnostik ermittelt werden. Bei pQE-VP26* liegt die Sensitivität mit dem errechneten cut off = 173 (Mittelwert der Negativseren + 3mal die Standardabweichung) bei 62% (n=13) und die Spezifität bei 100% (n=41) bzw. bei einem frei gewählten cut off von 150 die Sensitivität bei 77% und die Spezifität bei 95%.

Bei einer Bewertung im IgG-ELISA konnte bei pMAL-VP26* mit einem errechneten cut off von 210 (Mittelwert der Negativseren + 3mal die Standardabweichung) eine Sensitivität von 35% (n=49) und eine Spezifität von 100% (n=5) bzw. mit einem frei gewählten cut off von 100 eine Sensitivität von 59% und eine Spezifität von 80% für die IgG-Diagnostik ermittelt werden. Bei pQE-VP26* liegt die Sensitivität mit dem errechneten cut off von 215 (Mittelwert der Negativseren + 3mal die Standardabweichung) bei 53% und die Spezifität bei 100% bzw. mit dem frei gewählten cut off von 130 die Sensitivität bei 82% und die Spezifität bei 80%.

**Sequenz 1:** Aminosäurerest 1- 235 von VP26 (ORF23) (Stamm Ellen). Insgesamt 235 AS; Theoretisches Molekulargewicht 24,4 kDa. Der hervorgehobene Bereich entspricht dem immunreaktiven Fragment AS 12 - 235 von VP26* mit insgesamt 224 AS und einem theoretischen Molekulargewicht von 23 kDa. Die Raute (#) symbolisiert das Stop-Kodon. Die Numerierung der Aminosäuren beginnt beim Methionin (Startkodon) der publizierten Sequenz des ORF23 (A. J. Davison & J. E. Scott. (1986), J. Gen. Virol. 67, 1759-1816):

Met Thr Gln Pro Ala Ser Ser Arg Val Val Phe Asp Pro Ser Asn Pro
Thr Thr Phe Ser Val Glu Ala Ile Ala Ala Tyr Thr Pro Val Ala Leu
Ile Arg Leu Leu Asn Ala Ser Gly Pro Leu Gln Pro Gly His Arg Val
Asp Ile Ala Asp Ala Arg Ser Ile Tyr Thr Val Gly Ala Ala Ala Ser
Ala Ala Arg Ala Arg Ala Asn His Asn Ala Asn Thr Ile Arg Arg Thr
Ala Met Phe Ala Glu Thr Asp Pro Met Thr Trp Leu Arg Pro Thr Val
Gly Leu Lys Arg Thr Phe Asn Pro Arg Ile Ile Arg Pro Gln Pro Pro
Asn Pro Ser Met Ser Leu Gly Ile Ser Gly Pro Thr Ile Leu Pro Gln
Lys Thr Gln Ser Ala Asp Gln Ser Ala Leu Gln Gln Pro Ala Ala Leu
Ala Phe Ser Gly Ser Ser Pro Gln His Pro Pro Pro Gln Thr Thr Ser
Ala Ser Val Gly Gln Gln Gln His Val Val Ser Gly Ser Ser Gly Gln
Gln Pro Gln Gln Gly Ala Gln Ser Ser Thr Val Gln Pro Thr Thr Gly
Ser Pro Pro Ala Ala Gln Gly Val Pro Gln Ser Thr Pro Pro Pro Thr
Gln Asn Thr Pro Gln Gly Gly Lys Gly Gln Thr Leu Ser His Thr Gly
Gln Ser Gly Asn Ala Ser Arg Ser Arg Arg Val #

Die entsprechende Nukleotidsequenz ist in Tab. 1 dargestellt.

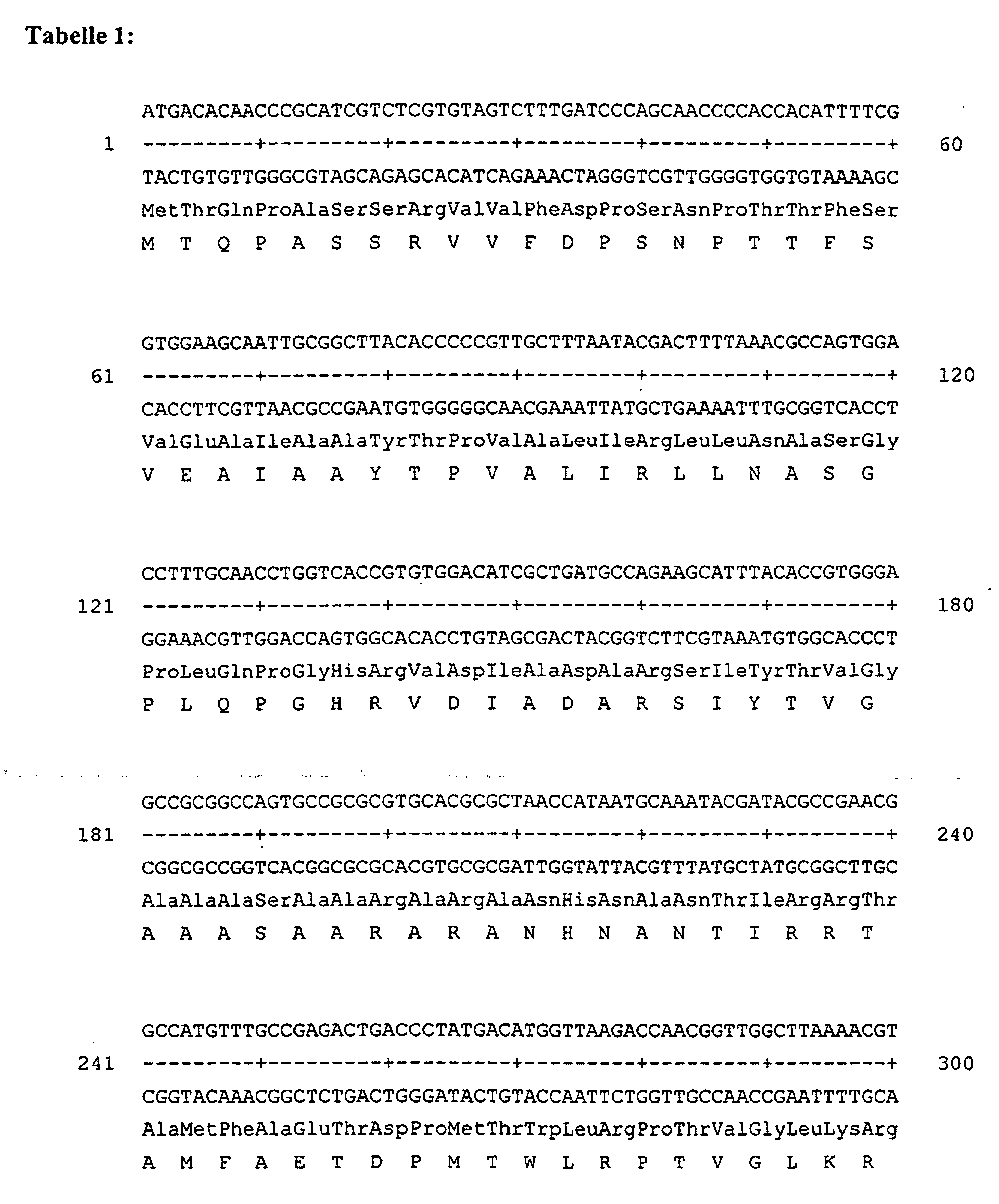

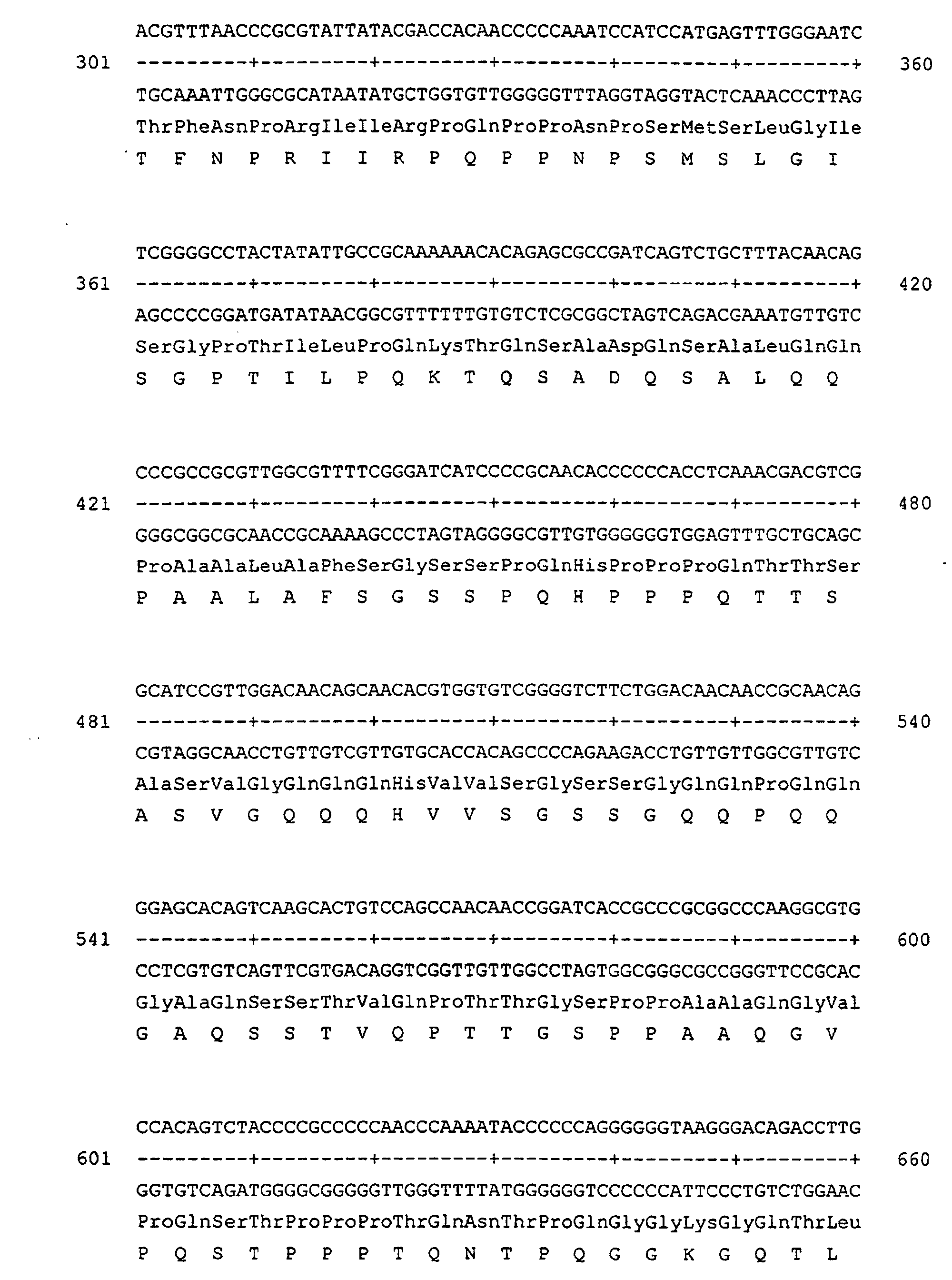

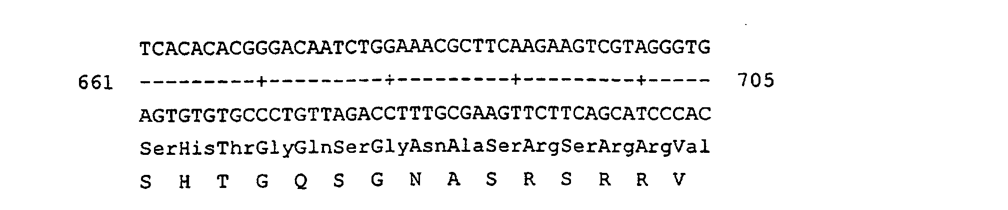

### Beispiele

### Beispiel 1:

### Gewinnung viraler VZV-Virionen und DNS-Extraktion:

Aus VZV-infizierten Fibroblasten wurden Virionen mittels Ultraschallbehandlung freigesetzt. Die Viren wurden über einen linearen Sucrose-Gradienten (20-70% (w/v)) von zellulären Bestandteilen gereinigt. Nach DNase I Inkubation wurde die virale DNS mittels Proteinase K und Sodiumdodecylsulfat (SDS) Behandlung aus den Virionen freigesetzt. Nach Phenol/Chloroform-Extraktion erfolgte die Fällung der viralen DNS mittels Ethanol.

### Beispiel 2:

### Anlegen einer genomische Expressionsbibliothek und Immunoscreening:

Die genomische VZV-DNS (siehe Beispiel 1) wurde durch Inkubation mit dem Restriktionsenzym SauIIIA partiell geschnitten und in die BamHI Schnittstelle der Lambda-DNS des ZAP-Express-Vektor-Systems (Stratagene) kloniert und in Lambda-Phagen verpackt. Nach Infektion von *E. coli.* (XL1-Blue MRF) konnte ein Phagentiter von 5x10⁵/ ml ermittelt werden. Durch Blau/ Weiß- Screening konnte festgestellt werden, daß 25% dieser Phagen keine inserierten DNS-Fragmente trugen. XL1-Blue-Zellen wurden mit den Phagen infiziert, in LB-Top-Agar überführt und auf LB-Agar-Platten für 3 Stunden bei 42°C inkubiert. Nach der Auflage von Isopropyl-β-D-Thiogalaktopyranosid getränkte Nitrozellulose-Membranen wurden die LB-Platten für 6-10 Stunden bei 37°C inkubiert. Anschließend wurden die Membranen mittels Immunofärbung unter Verwendung eines Pool-Serums aus 25 VZV-reaktiven, humanen Seren als Erstantikörper und einen Peroxidasegekoppelten Kaninchen anti- Human IgG Zweitantikörper auf immunreaktive Klone analysiert. Immunreaktive Klone wurden einer Plaquereinigung unterzogen. Der den Phagemidvektor pBK/CMV kodierende Bereich inklusive inserierter VZV-DNS wurde mittels ExAssist-Helfer-Phagen-System (Stratagene) ausgeschnitten und zirkularisiert. Der VZV-DNS-Anteil wurde mittels Sequenzierung analysiert und durch Datenbanksuche (GCG-Programm Blastn) identifiziert.

### Beispiel 3:

### Klonierung des ORF23 (VP26):

Die virale DNS (siehe Beispiel 1) bzw. der aus dem immunreaktiven Lambda-Phagen gewonnene Phagemidvektor pBK/CMV-23 dienten als Matrize für die Amplifikation des ORF23 (VP26) bzw. des ORF23-Fragments (VP26*), das im Vektor pBK/CMV-23 inseriert war. Als Amplifikationsoligonukleotide dienten folgende Primer: 
VP26:   VP26H 5' GGAATTCCGGATGACACAACCCGCATCGTCTCGTGTA 3';
        VP26R 5' GCTCTAGATTACACCCTACGACTTCTTGAAGCGTTTCC 3'.
 
VP26*:  VP26*H 5' GGAATTCCGCGCCTGCAGGTCGACACTAGTGGAT 3'
        VP26*R 5' GCTCTAGATTACACCCTACGACTTCTTGAAGCGTTTCC 3'.
 Diese Oligonukleotide sind dem entsprechenden Sequenzabschnitt der publizierten VZV-Sequenz (A. J. Davison & J. E. Scott. (1986), J. Gen. Virol. 67, 1759-1816) bzw. der Sequenz des Vektors pBK/CMV (Stratagene) komplementär, enthalten aber an ihren 5' Termini eine Restriktionsschnittstellensequenz, die nicht mit der Matrizen-DNS hybridisierte. Nach erfolgter Amplifikation wurde das 726 bp bzw. 714 bp große Amplifikat mit den Restriktionsenzymen EcoRI und XbaI terminal geschnitten und in den zuvor mit EcoRI und XbaI linearisierten Expressionsvektor pMAL-c2 ligiert . Der gesamte ORF23 wurde in überlappender bidirektionaler Weise vollständig sequenziert. Die Vektoren wurden als pMAL-VP26 bzw. pMAL-VP26* bezeichnet.
Ferner wurde der das immunreaktive Protein kodierende Bereich (VP26*) in den Vektor pQE30 kloniert. Als Matrizen-DNS diente genomische VZV-DNS. Als Amplifikationsoligonukleotide dienten folgende Primer: 
VP26*:  VP26* 5' CGGATCCGATCCCAGCAACCCCACCAC 3';
        VP26R 5' GCTCTAGATTACACCCTACGACTTCTTGAAGCGTTTCC 3'.
 Diese Oligonukleotide sind dem entsprechenden Sequenzabschnitt der publizierten VZV-Sequenz (A. J. Davison & J. E. Scott. (1986), J. Gen. Virol. 67, 1759-1816) komplementär, enthalten aber an ihren 5' Termini eine Restriktions-schnittstellensequenz, die nicht mit der Matrizen-DNS hybridisierte. Nach erfolgter Amplifikation wurde das 690 bp große Amplifikat mit dem Restriktionsenzym EcoRI terminal geschnitten und in den zuvor mit EcoRI und SmaI linearisierten Expressionsvektor pQE30 ligiert. Der Vektor wurde als pQE-VP26* bezeichnet.

### Beispiel 4

### a) Herstellung der rekombinanten Proteine pMal-VP26* und pQE-VP26*:

Die Expression und Reinigung des rekombinanten Proteins pMAL-VP26* erfolgte durch Affinitätschromatographie entsprechend den Angaben des Herstellers (New England Biolabs, 800-21S).
Die Expression und Reinigung des rekombinanten Proteins pQE-VP26* erfolgte durch Metal Affinity Chromatography unter nativen Bedingungen nach Angaben des Herstellers (Fa. Clontech, Talon Metal Affinity Resin, PT1320-1).

### b) Herstellung der Festphase I (erfindungsgemäßes System) pMal-VP26* oder pQE- VP26*:

Mikrotitrationsplatten Typ B (Fa. Greiner) wurden mit 110µl je Vertiefung Beschichtungslösung (1 µg/ml rekombinantes pMal-VP26* bzw. 1µg/ml rekombinantes pQE-VP26* in 50 mM Natriumcarbonat-Puffer, pH 9.5) für 18 Stunden bei 4°C inkubiert. Die Vertiefungen der Mikrotitrationsplatten wurden dann dreimal mit je 300 µl Waschlösung (50mMTris/EDTA pH 7,0-7,4 + 0,5% BSA) gewaschen.
Für die Austestung im Anti- VZV IgG Enzymimmunoassay wurde das rekombinante Protein pQE-VP26* unter gleichen Bedingungen wie oben beschrieben, aber mit einer Konzentration von 2 µg/ml, beschichtet.

### c) Enzymimmunoassay zum Nachweis von VZV-IgM Antikörpern:

Der Enzymimmunoassay zum Nachweis von Anti-VZV IgM wurde wie folgt durchgeführt: Die Seren wurden zuvor 1:84 in POD Probenpuffer (Behring Diagnostics, OWBE 945) verdünnt und anschließend für 15 Minuten 1:2 mit RF Adsorbens (Behring Diagnostics, OUCG 945) bei Raumtemperatur vorinkubiert. In die Vertiefungen der nach Beispiel 4b hergestellten Mikrotitrationsplatten wurden je 100 µl der wie oben beschriebenen vorverdünnten Seren für 1h bei 37°C inkubiert. Nach 3maligem Waschen mit Waschlösung POD (Behring Diagnostics, OSEW 965) wurden 100 µl des Konjugates Anti-Human IgM/POD (Behring Diagnostics, Ch. 4241313), 1:25 für pQE-VP26* und 1:50 für pMalVP26*, verdünnt in Microbiol-Konjugatpuffer (Behring Diagnostics, OUWW 935), einpipettiert. Mit 3 weiteren Waschschritten wurde die 1 stündige Inkubation (+37°C) beendet. Die gebundene Peroxidase-Aktivität, die direkt mit der Anzahl der gebundenen VZV-Antikörper korreliert, wurde durch Zugabe von H₂O₂/ Tetramethylbenzidin (Behring Diagnostics, OUVG 945) bestimmt. Der Substratumsatz wurde nach 30 Minuten bei Raumtemperatur durch Zugabe von 0.5 M Schwefelsäure (Behring Diagnostics, OSFA 965) gestoppt und die Extinktion bei 450 nm gemessen.
Anti-VZV positive sowie anti-VZV negative Seren wurden sowohl im Referenzsystem Enzygnost Anti VZV/IgM (Behring Diagnostics, OWLW 155), wie auch im erfindungsgemäßen Enzymimmunoassay untersucht.
Die Resultate (Extinktionseinheiten) der Untersuchung finden sich in der Tabelle 2.

Bei einer Bewertung im ELISA konnte bei pMAL-VP26* mit einem errechneten cut off = 187 (Mittelwert der Negativseren + 3mal die Standardabweichung) eine Sensitivität von 69% (n=13) und eine Spezifität von 98% (n=41) bzw. mit einem frei gewählten cut off von 120 eine Sensitivität von 85% und eine Spezifität von 95% für die IgM-Diagnostik ermittelt werden. Bei pQE-VP26* liegt die Sensitivität mit dem errechneten cut off = 173 (Mittelwert der Negativseren + 3mal die Standardabweichung) bei 62% (n=13) und die Spezifität bei 100% (n=41) bzw. bei einem frei gewählten cut off von 150 die Sensitivität bei 77% und die Spezifität bei 95%.

### Beispiel 5

### a) Enzymimmunoassay zum Nachweis von VZV-IgG Antikörpern:

Für den Nachweis von Anti VZV IgG im Enzymimmunoassay wurden je 100 µl der 1:100 vorverdünnten Seren in POD Probenpuffer (Behring Diagnostics, OWBE 945) in der nach Beispiel 4b hergestellten Mikrotiterplatte einpipettiert und für 1h bei 37°C inkubiert.
Nach 3maligem Waschen mit Waschlösung POD (Behring Diagnostics, OSEW 965) wurden 100 µl des Konjugates Anti-Human IgG/POD (Behring Diagnostics Ch. 243713), 1:50 verdünnt in Microbiol-Konjugatepuffer (Behring Diagnostics, OUWW 935), einpipettiert. Mit drei weiteren Waschschritten wurde die 1stündige Inkubation (+37°C) beendet. Die gebundene Peroxidase-Aktivität, die direkt mit der Anzahl der gebundenen VZV-Antikörper korreliert, wurde durch Zugabe von H₂O₂/ Tetramethylbenzidin (Behring Diagnostics, OUVG 945) bestimmt. Der Substratumsatz wurde nach 30 Minuten bei Raumtemperatur durch Zugabe von 0.5 M Schwefelsäure (Behring Diagnostics, OSFA 965) gestoppt und die Extinktion bei 450 nm gemessen.

Anti-VZV positive sowie anti-VZV negative Seren wurden sowohl im Referenzsystem Enzygnost Anti VZV/IgG (Behring Diagnostics, OWLT 155), wie auch im erfindungsgemäßen Enzymimmunoassay untersucht. Die Resultate (Extinktionseinheiten) der Untersuchung finden sich in der Tabelle 3.

Bei einer Bewertung im ELISA konnte bei pMAL-VP26* mit einem errechneten cut off von 210 (Mittelwert der Negativseren + 3mal die Standardabweichung) eine Sensitivität von 35% (n=49) und eine Spezifität von 100% (n=5) bzw. mit einem frei gewählten cut off von 100 eine Sensitivität von 59% und eine Spezifität von 80% für die IgG-Diagnostik ermittelt werden. Bei pQE-VP26* liegt die Sensitivität mit dem errechneten cut off von 215 (Mittelwert der Negativseren + 3mal die Standardabweichung) bei 53% und die Spezifität bei 100% bzw. mit dem frei gewählten cut off von 130 die Sensitivität bei 82% und die Spezifität bei 80%.

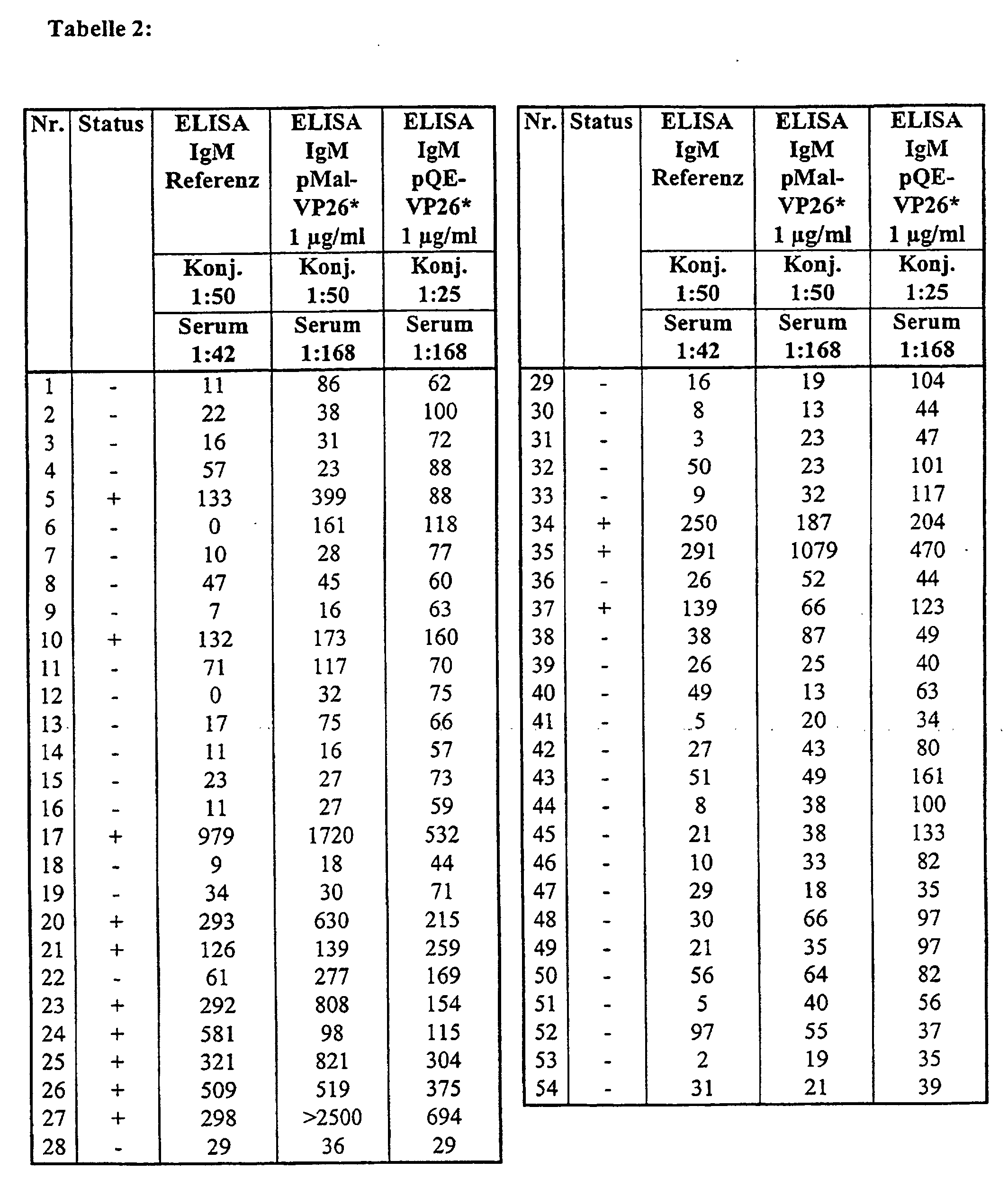

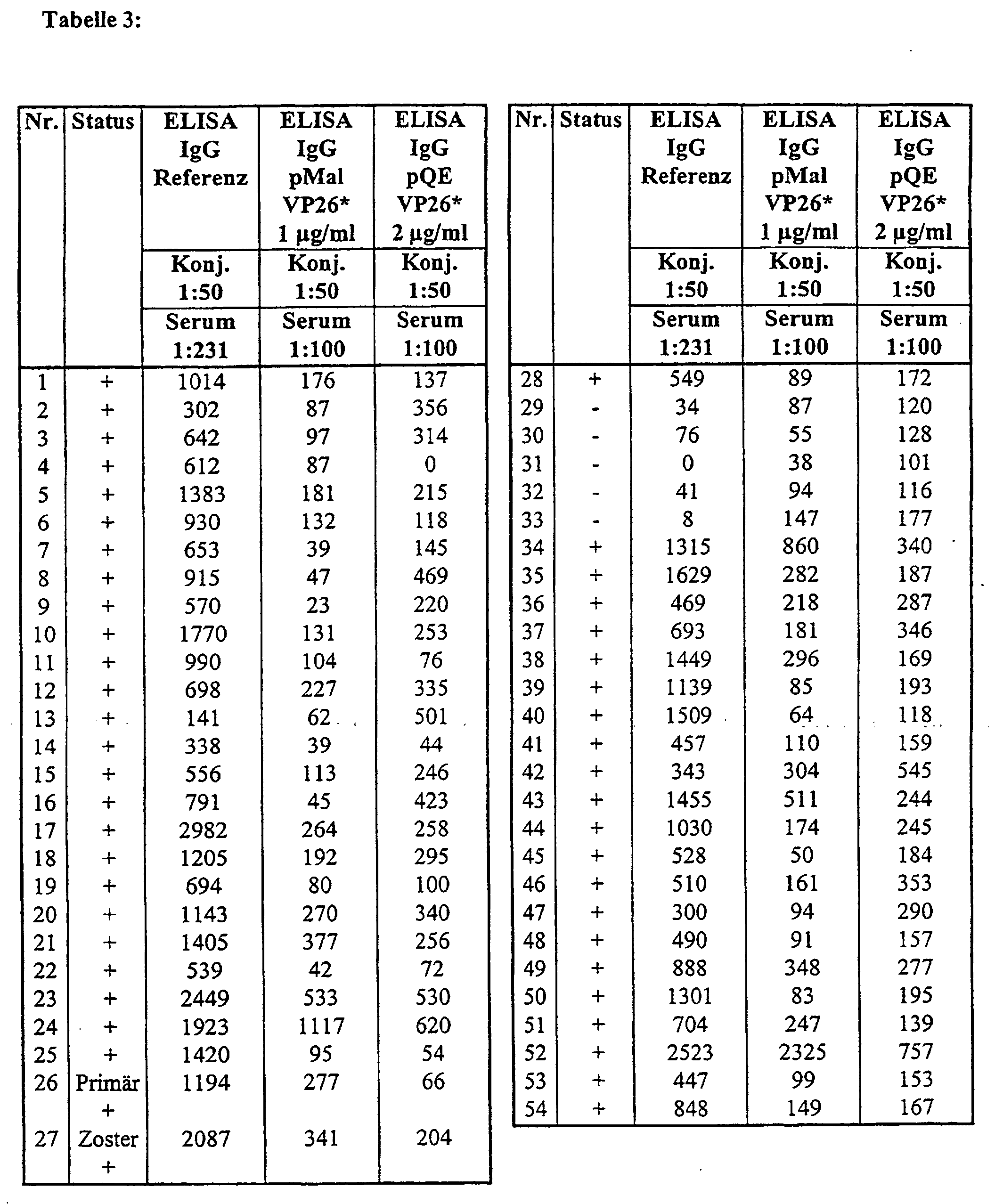

## Patentansprüche

1. Immunreaktives Peptid, **dadurch gekennzeichnet, daß** es aus den AS 12 - 235 des VP 26 des Varicella-Zoster-Virus (VZV) gemäß Tabelle 1 besteht sowie Varianten dieses Peptids, **dadurch gekennzeichnet, daß** die Varianten natürlicherweise variierende AS-Sequenzen aufweisen, die sich aufgrund von VZV-Stammvariationen ergeben.

2. Immunreaktives Peptid, dessen AS-Sequenz mit der AS-Sequenz der Position 12 bis 235 gemäß Tabelle 1 identisch ist.

3. Nukleinsäure, kodierend für ein immunreaktives Peptid mit der Aminosäuresequenz gemäß Anspruch 1 oder 2.

4. Nukleinsäure, die mit der Nukleinsäure gemäß Anspruch 3 unter stringenten Bedingungen hybridisiert und für ein immunreaktives Peptid gemäß Anspruch 1 oder 2 kodiert, welches von gegen VZV gerichteten Antikörpern, nicht jedoch von gegen andere Herpesviren gerichteten Antikörpern erkannt wird.

5. Immunchemisches Verfahren zum Nachweis von Antikörpern gegen VZV in einer Probe mittels eines immunreaktiven Peptids gemäß Anspruch 1 oder 2.

6. Verwendung der Nukleinsäure gemäß Ansprüche 3 oder 4 zum Nachweis von VZV mittels Nukleinsäurehybridisierung.

7. Testbesteck zum Nachweis von Antikörpern gegen VZV, enthaltend ein immunreaktives Peptid gemäß Anspruch 1 oder 2.

8. Testbesteck zum Nachweis von VZV, enthaltend die Nukleinsäure gemäß Anspruch 3 oder 4.

## Claims

1. An immunoreactive peptide which is composed of AA 12-235 of varicella zoster virus (VZV) VP26 as depicted in Table 1 and also variants of this peptide, with these variants possessing AA sequences which vary naturally and which result from VZV strain variations.

2. An immunoreactive peptide whose AA sequence is identical to the AA sequence from position 12 to 235 as depicted in Table 1.

3. A nucleic acid which encodes an immunoreactive peptide having the amino acid sequence as claimed in claim 1 or 2.

4. A nucleic acid which hybridizes under stringent conditions with the nucleic acid as claimed in claim 3 and encodes an immunoreactive peptide as claimed in claim 1 or 2 which is recognized by antibodies which are directed against VZV but not by antibodies which are directed against other herpes viruses.

5. An immunochemical method for detecting antibodies against VZV in a sample using an immunoreactive peptide as claimed in claim 1 or 2.

6. The use of the nucleic acid as claimed in claim 3 or 4 for detecting VZV by means of nucleic acid hybridization.

7. A test kit for detecting antibodies against VZV, which comprises an immunoreactive peptide as claimed in claim 1 or 2.

8. A test kit for detecting VZV, which comprises the nucleic acid as claimed in claim 3 or 4.

## Revendications

1. Peptide immunoréactif **caractérisé en ce qu'**il est composé des AA 12-235 de la protéine VP26 du virus varicelle-zoster (VZV) selon le tableau 1, et variantes de ce peptide, **caractérisées en ce qu'**elles présentent des séquences AA variant naturellement en raison des variations des souches VZV.

2. Peptide immunoréactif, dont la séquence AA est identique à la séquence AA des positions 12 à 235 selon le tableau 1.

3. Acide nucléique codant pour le peptide immunoréactif présentant la séquence d'acides aminés selon les revendications 1 ou 2.

4. Acide nucléique qui s'hybride à l'acide nucléique selon la revendication 3 dans des conditions stringentes et qui code pour un peptide immunoréactif selon les revendications 1 ou 2, qui est reconnu par les anticorps dirigés contre le virus VZV, mais pas par les anticorps dirigés contre les autres herpesvirus.

5. Procédé immunochimique pour la détection d'anticorps contre le virus VZV dans un échantillon au moyen d'un peptide immunoréactif selon les revendications 1 ou 2.

6. Utilisation de l'acide nucléique selon les revendications 3 ou 4 pour la détection du virus VZV par hybridation de l'acide nucléique.

7. Trousse d'essai pour la détection d'anticorps contre le virus VZV contenant un peptide immunoréactif selon les revendications 1 ou 2.

8. Trousse d'essai pour la détection du virus VZV contenant l'acide nucléique selon les revendications 3 ou 4.
